# EUROPÄISCHE PATENTANMELDUNG

(11) **EP 0 765 669 A1**
(43) Veröffentlichungstag der Anmeldung: **02.04.1997**
(21) Anmeldenummer: 96111052.5
(22) Anmeldetag: 09.07.1996
(51) Int. Cl.: A61K 38/48, C12N 9/64

(54) **Pharmazeutische Zusammensetzung zur Behandlung von Blutgerinnungsstörungen, Verfahren zur Herstellung derselben und deren Verwendung**

(30) Priorität: 28.08.1995 DE 19531637
(71) Anmelder: IMMUNO AG, 1221 Wien (AT)
(72) Erfinder: Turecek, Peter, Dr., 3400 Klosterneuburg (AT); Schwarz, Hans-Peter, 1180 Wien (AT); Redl, Gerda, Dr., 2301 Rutzendorf (AT)
(74) Vertreter: Kolb, Helga, Dr. Dipl.-Chem.

(57) **Zusammenfassung**

Die Erfindung betrifft eine pharmazeutische Zusammensetzung zur Behandlung von Patienten mit Blutgerinnungsstörungen, die durch einen Gerinnungsfaktor-Mangel bzw. Inhibitoren von Gerinnungsfaktoren bedingt sind, wobei die Zusammensetzung eine FEIB-Aktivität aufweist und dadurch gekennzeichnet ist, dass sie den Faktor VIIa und mindestens einen weiteren Wirkstoff und die Aktivität von mindestens 10 Faktor VIIa-Einheiten pro Einheit FEIBA aufweist. Ausserdem umfasst die Erfindung ein Verfahren zur Herstellung der pharmazeutischen Präparation und deren Verwendung.

## Beschreibung

Die Erfindung betrifft eine pharmazeutische Zusammensetzung zur Behandlung von Blutgerinnungsstörungen, insbesondere von Faktor VIII Inhibitor-Patienten. Die Erfindung betrifft weiters ein Verfahren zur Herstellung einer derartigen Zusammensetzung sowie deren Verwendung.

Die Blutgerinnung wird durch eine Reihe von aufeinanderfolgenden Reaktionen verschiedener Proteine bzw. Enzyme ausgelöst. Durch einen Mangel an Blutgerinnungsfaktoren wird die Bildung von Fibrin aus Fibrinogen und damit der Wundverschluss verhindert; die Folge sind Blutungen. Ein solcher Fall liegt bei der Hämophilie A vor. Diese ist die am meisten verbreitete Blutungskrankheit und wird durch den Mangel an Faktor VIII hervorgerufen. Zur Substitutionsbehandlung der Hämophilie A werden Präparate eingesetzt, die den Faktor VIII enthalten. Die Behandlung mit diesen Präparaten führt in den meisten Fällen zu einer raschen Blutstillung.

Es gibt jedoch auch Patienten, bei denen nicht nur ein Mangel an Faktor VIII auftritt, sondern die auch einen gegen Faktor VIII gerichteten Hemmstoff (Inhibitor) entwickelt haben. Ein weiteres Patientenkollektiv weist Faktor VIII-Inhibitoren auf, ohne an Hämophilie A zu leiden. Je nach vorhandener Menge an Faktor VIII-Inhibitoren wird die Wirkung von zugeführtem Faktor VIII durch dessen Neutralisierung inhibiert.

Zur Behandlung von Faktor VIII-Inhibitor-Patienten werden derzeit Präparate auf Basis einer Plasmafraktion angeboten, die ein Gemisch von Gerinnungsfaktoren enthält. Diese Plasmafraktion kann beispielsweise die Faktoren des Prothrombinkomplexes (Faktor II, VII, IX und X) enthalten. Eine blutgerinnungsfördernde Präparation mit Faktor VIII-Inhibitor-Bypass-Aktivität (FEIBA® TIM 4, Fa. Immuno AG) wird beispielsweise gemäss AT-0 368 883 durch eine Behandlung von Kryoüberstand erhalten. Diese Präparation enthält ebenfalls die Gerinnungsfaktoren II, VII, IX und X.

Die Wirkung eines FEIBA-Präparates ist aufgrund dessen komplexer Zusammensetzung vielschichtig. Mariani et al (Thrombosis Res. 31, 475-488, (1983)) nennt als ein Wirkungsprinzip den Faktor VII in seiner aktivierten Form. Es wurde festgestellt, dass nach Infusion einer FEIBA-Präparation ein erhöhter Gehalt von Faktor VIIa im Plasma Hämophiler auftritt.

Ebenso wird von Teitel (Thrombosis and Haemostasis 66 (5) 559-564, (1991)) die Rolle von Faktor VIIa in Prothrombinkomplex-Konzentraten mit einer "Faktor VIII-Bypassing-Aktivity" diskutiert. Gleichzeitig wird auch auf das Wirkungsprinzip von Faktor Xa in derartigen Präparaten eingegangen. Die untersuchten Prothrombinkomplex-Konzentrate enthielten Faktor VIIa, ausgedrückt durch das Verhältnis der Faktor VII-Aktivität zu Faktor VII-Antigen von 2,1 und 2,5.

Die gemäss EP-0 044 343-B1 hergestellte Prothrombinhaltige therapeutische Zusammensetzung ist zur Behandlung von Gerinnungsfaktor-Inhibitoren geeignet und enthält einen aktivierten Prothrombinkomplex, bei dem die Faktoren teilweise aktiviert sind. Der Anteil an Faktor VIIa liegt bei 8-80 Einheiten/ml. Die Faktor IX-Konzentration liegt im Bereich von 15 bis 112 Einheiten/ml. Entsprechend beträgt der Gehalt an Faktor VIIa, bezogen auf Faktor IX, 0,07 - 5,3 E Faktor VIIa/E Faktor IX.Vinazzer (Thromb. Res. 26:21-29 (1982)) zeigt den Unterschied der Präparate AUTOPLEX, welches gemäss EP-44 343 hergestellt wird, und FEIBA. Wie dort gezeigt wird, zeichnet sich AUTOPLEX durch den höheren Gehalt an Thrombin (Faktor IIa), gemessen in NIH-Units, im Vergleich zu FEIBA aus (siehe Tabelle 1, Seite 24).

Aber auch hochgereinigte Faktor VIIa-Präparationen werden zur Therapie von Gerinnungsfaktor-Inhibitorzuständen vorgeschlagen (z.B. EP 0 082 182-B1) und Hedner et al. (Haemostasis 19, 335-343 (1989)).

Ein Verfahren zur Herstellung eines Konzentrates, welches den hochgereinigten Faktor VIIa enthält, ist auch in EP-0 547 932-A1 beschrieben. Aus Kryoüberstand wird durch Anionenaustauschchromatographie der Faktor VII von den Gerinnungsfaktoren II, IX und X abgetrennt. Der Faktor VII wird dann einer weiteren Reinigung und Aktivierung unterzogen, wobei die Zugabe von exogenen Proteinen vermieden wird. Anschliessend wird der Faktor VIIa durch eine Behandlung mit einem organischen Lösungsmittel/Detergens (TnBP/Tween) zur Inaktivierung von Viren behandelt.

Bei der Verwendung von Plasma oder einer Plasmafraktion als Ausgangsmaterial zur Herstellung pharmazeutischer Präparate besteht das Risiko der Übertragung von infektiösen Agenzien. Trotz Spenderauswahl und Testen der Einzelspenderplasmen auf möglicherweise vorhandene Viren, wie HIV, Hepatitis B- oder Hepatitis C-Virus, besteht die Möglichkeit, dass ein Plasmapool infektiös ist. Diese restliche Infektiösität ist aufgrund einer limierten Empfindlichkeit der Testsysteme aber auch aufgrund der Inkubationszeit bis zum Auftreten bestimmbarer Infektionsmarker (diagnostisches Fenster) nicht auszuschliessen.

Pharmazeutische Präparate, die aus Plasma oder einer Plasmafraktion hergestellt sind, werden daher diversen Behandlungen zur Inaktivierung von potentiell vorhandenen Viren unterzogen. Ein bewährtes Verfahren zur Inaktivierung von sowohl membranumhüllten als auch nicht-membranumhüllten Viren ist in EP-0 159 311-B1 beschrieben. Dabei wird eine pharmazeutische Präparation in festem Zustand nach Einstellen eines Wassergehaltes auf einen Wert von 5-70% erhitzt. Diese Behandlung wird auch als Dampfbehandlung beschrieben. Anstelle von Wasser kann auch Methanol oder Ethanol verwendet werden.

Ebenso sind aber Kombinationen von Virusinaktivierungsmethoden möglich. Gemäss der EP 0 519 901 wird eine Behandlung mit hochkonzentrierten Tensiden mit einer Hitzebehandlung kombiniert.

Aufgabe der Erfindung ist es, eine verbesserte pharmazeutische Zusammensetzung zur Behandlung von Blutgerinnungsstörungen, insbesondere von Faktor VIII-Inhibitor-Patienten sowie ein einfaches Verfahren zu dessen Herstellung zur Verfügung zu stellen.

Diese Aufgabe wird erfindungsgemäss durch eine pharmazeutische Zusammensetzung mit FEIB-Aktivität gelöst, welche den Faktor VIIa und mindestens einen weiteren Wirkstoff enthält und die Aktivität von 10 Faktor VIIa-Einheiten pro Einheit FEIBA aufweist.

Der Gehalt an Faktor VIIa in der erfindungsgemässen Zusammensetzung beträgt mindestens 10, vorzugsweise 10 bis 100, am meisten bevorzugt 10 bis 20, insbesondere 10 bis 15 Einheiten pro Einheit FEIB-Aktivität. Die FEIB-Aktivität wird mittels einer Methode gemäss AT-350 726 bestimmt

Es hat sich herausgestellt, dass ein Präparat, welches den hochgereinigten Faktor VIIa als einzige effektive Substanz enthält, keine FEIB-Aktivität besitzt. Es war daher überraschend, dass ein Zusatz von Faktor VIIa zu einem Präparat mit FEIB-Aktivität, wie z.B. einer den aktivierten Prothrombinkomplex-haltigen Fraktion, zu einer wesentlichen Verbesserung der Wirksamkeit einer pharmazeutischen Präparation zur Behandlung von Blutgerinnungsstörungen, insbesondere von Faktor VIII-Inhibitor-Patienten beiträgt.

Der Gehalt an Faktor VIIa wurde in den nachfolgenden Beispielen mit der von van Deijk (Haemostasis 13: 192-197, 1983) und der in EP-0 0547 932 beschriebenen Methode mit bovinem Thromboplastin bestimmt. Ausserdem kann Faktor VIIa auch unter Verwendung von rekombinantem, löslichem *Tissue Factor* nach der Methode von Morrissey et al. (Blood 81: 734-744, (1993)) und WO 9218870 bestimmt werden.

Als Wirkstoffe in einer Präparation von FEIB-Aktivität sind die Gerinnungsfaktoren II, IX und X sowie ausserdem z.B. auch *Tissue Factor* oder Xa und Phospholipide zu nennen. Die vorstehende Aufzählung von Substanzen in einer Präparation mit FEIB-Aktivität ist nicht vollständig und soll daher auch nicht als limitierend gewertet werden.

Die erfindungsgemässe Präparation enthält vorzugsweise die Gerinnungsfaktoren II, IX und X, und zwar vorzugsweise in einer Konzentration, die für jeden Faktor einem Verhältnis von 0.5 - 2, besonders bevorzugt 0,5 - 1,5 E/E FEIBA entspricht. Die Bestimmung erfolgt gemäss Testbeschreibung in AT-350 726.

Damit unterscheidet sich die erfindungsgemässe Zusammensetzung von den bekannten Präparaten nicht nur in ihrem aussergewöhnlich hohem Verhältnis von Faktor VIIa zu FEIBA, sondern auch in ihrem Verhältnis von Faktor VIIa zu Faktor IX. Letzteres ist beispielsweise deutlich höher als in EP- 0 044 343.

Es ist weiters vorteilhaft, wenn das erfindungsgemässe Präparat zusätzlich Protein C und/oder Protein S enthält.

Es war überraschend, dass die erfindungsgemässe Präparation in einfacher und ökonomischer Weise hergestellt werden kann. Die Erfindung umfasst deshalb auch ein Herstellungsverfahren, das durch Kontaktieren einer Fraktion, wie vorstehend erläutert, mit einem Anionenaustauscher gekennzeichnet ist, wobei der Gerinnungsfaktor VII zumindest teilweise aktiviert wird. Dabei werden Bedingungen gewählt, die die gleichzeitige Adsorption von Faktor VII bzw. Faktor VIIa mit den Gerinnungsfaktoren II, IX und X zulassen, beispielsweise unter Verwendung von Puffern geringer Ionenstärke. Anschliessend wird der Anionenaustauscher abgetrennt, z.B. durch Sedimentation, und die Fraktion, welche die Gerinnungsfaktoren II, VIIa, IX und X enthält, gewonnen. Hierbei besitzt die Fraktion bereits vor dem Kontaktieren eine FEIBA, bzw. es wird eine FEIBA währenddessen in der Fraktion generiert.

Als Anionenaustauschmaterial wird vorzugsweise ein starker Anionenaustauscher, beispielsweise eine Matrix mit quaternären Ammoniumgruppen, wie QAE-, TMAE- und Q-Gruppen, verwendet. Als Träger für die Anionenaustauschgruppen wird vorzugsweise quervernetztes Dextran, wie Sephadex oder Sepharose, oder synthetisches Material, wie Fraktogel, verwendet.

Dieses Herstellungsverfahren zeichnet sich u.a. durch seine ökonomische Vorgehensweise aus. Das Verfahren hat den Vorteil, dass der Gerinnungsfaktor VII nicht erst abgetrennt werden muss, um dann wieder der Präparation zugesetzt zu werden. Ausserdem wird in einem Schritt die Gerinnungsfaktor-haltige Fraktion nicht nur gereinigt, sondern auch der Gerinnungsfaktor VII aktiviert. Gleichzeitig ist auch die teilweise Aktivierung von Faktor IX und Faktor X, nicht aber von Faktor II, gegeben.

Die erfindungsgemässe Zusammensetzung ist auch erhältlich durch Vereinigen einer Fraktion, enthaltend einen gereinigten Prothrombinkomplex, und einer Fraktion, enthaltend den gereinigten Faktor VIIa, und durch eine Behandlung zur Inaktivierung von Viren. Die erfindungsgemässe pharmazeutische Zusammensetzung kann aber auch aus einer Fraktion, enthaltend einen gereinigten partiellen Prothrombinkomplex (Faktoren II, IX und X), und einer Fraktion, enthaltend den gereinigten Faktor VIIa, zusammengesetzt sein, wobei sie zur Inaktivierung von Viren behandelt wird. Der zur Herstellung der erfindungsgemässen Zusammensetzung verwendete Prothrombinkomplex liegt vorzugsweise als aktivierter Prothrombinkomplex oder als FEIBA-Präparation vor. In einem aktivierten Prothrombinkomplex sind die Gerinnungsfaktoren VII, IX und X teilweise aktiviert. Die Aktivierung des Faktor II wird jedoch in einem solchen Präparat vermieden, um eine unmittelbare thrombogene Aktivität auszuschliessen.

Bei Verwendung bestimmter Bedingungen, wie hohe Ionenstärke in der Ausgangslösung bzw. im Waschpuffer und starke Anionenaustauschergele, z.B. Q-Sepharose Fast Flow (Fa. Pharmacia), kann aktivierter Prothrombinkomplex mit hoher Ausbeute aus Plasma oder dem Plasmaüberstand nach Abtrennung des Kryopräzipitates als FEIBA-Präparation isoliert werden. Derartige Gele binden dann relativ wenig Faktor VII oder VIIa. Daher kann aus demselben Ausgangsmaterial mit hoher Ausbeute in einem weiteren Verfahren Faktor VIIa isoliert werden.

Die erhaltenen Fraktionen werden auf übliche Weise zu pharmazeutischen Präparationen z.B. durch Zusetzen von pharmazeutisch akzeptablen Trägern bzw. Hilfsstoffen, Sterilfiltrieren und Lyophilisieren zu lagerfähigen pharmazeutischen Präparationen verarbeitet.

Die erfindungsgemässe Zusammensetzung kann durch ein weiteres Verfahren mit den folgenden Schritten hergestellt werden:
a) Isolieren der Gerinnungsfaktoren II, IX und X als Einzelkomponenten oder als Gemisch aus einem oder mehreren Ausgangsmaterialien enthaltend einen oder mehrere der Gerinnungsfaktoren II, IX bzw. X ,
b) Isolieren des Gerinnungsfaktors VII/VIIa aus einem Ausgangsmaterial enthaltend den Gerinnungsfaktor VIIa bzw. den Gerinnungsfaktor VII, wobei der Gerinnungsfaktor VII zumindest teilweise aktiviert wird,
c) Vorbehandeln oder Behandeln der genannten Ausgangsmaterialien oder der isolierten Gerinnungsfaktoren zur Inaktivierung von infektiösen Agenzien, und
d) Mischen der isolierten Gerinnungsfaktoren zusammen mit einem pharmazeutisch akzeptablen Träger.

In einer bevorzugten Ausführungsform wird der Faktor VIIa zum Prothrombinkomplex, der auch als partieller Prothrombinkomplex vorliegen kann, nach Reinigung der Komponenten mit einem pharmazeutisch akzeptablen Träger zusammengemischt und anschliessend einer Behandlung zur Virusinaktivierung, beispielsweise einer Hitzebehandlung, unterzogen.

Der Zusatz bzw. der Gehalt an hochreinem Faktor VIIa ist besonders vorteilhaft, weil somit weniger Fremdprotein als Verunreinigung in das Präparat eingebracht wird. Die spezifische Aktivität des Faktor VIIa in dem Präparat beträgt vorzugsweise mehr als 10 E/mg bis zu 1000 E/mg Protein.

Bevorzugt liegt der Prothrombinkomplex als aktivierter Prothrombinkomplex oder FEIBA-Präparation vor.

Zur Inaktivierung von infektiösen Agenzien (Viren) wird vorzugsweise eine Hitzebehandlung, z. B. eine Dampfbehandlung, durchgeführt. Dabei hat sich herausgestellt, dass eine Hitzebehandlung der Gerinnungsfaktoren in festem Zustand ohne Zusatz von stabilisierenden Proteinen, wie Albumin, durchgeführt werden kann. Der Faktor VIIa ist überraschenderweise so stabil, dass er auch in gereinigtem Zustand zur Inaktivierung von infektiösen Agenzien behandelt werden kann.

Die Hitzebehandlung zur Inaktivierung von Viren hat den Vorteil , dass nicht nur lipidumhüllte Viren inaktiviert werden, sondern auch Viren, die nicht mit einer Lipidhülle umgeben sind.

Das erfindungsgemässe Verfahren zeichnet sich dadurch aus, dass der Faktor VIIa aus einer Fraktion gewonnen werden kann, die bei der Herstellung einer Fraktion, enthaltend den (partiellen) Prothrombinkomplex, als Nebenprodukt anfällt. Daher zeichnet sich auch dieses Verfahren besonders durch seine Wirtschaftlichkeit aus.

Mit den erfindungsgemässen Verfahren ist es möglich, alle Komponenten der erfindungsgemässen Zusammensetzung aus einem einzigen Ausgangsmaterial, vorzugsweise einem Plasmapool oder einer Plasmafraktion, zu erhalten. Damit wird die Sicherheit der erfindungsgemäss erhaltenen pharmazeutischen Zusammensetzung erhöht.

Das erfindungsgemässe Verfahren sieht in einer bevorzugten Ausführungsform die Isolierung des Gerinnungsfaktors VII durch chromatographische Methoden, vorzugsweise mit Hilfe von Anionenaustauschern, wie z.B. DEAE-Sephacel®, DEAE-Sephadex®, DEAE-Sepharose® CL6B, DEAE-Sepharose® Fast Flow, QAE-Sephadex®, Q-Sepharose® Fast Flow, Q-Sepharose® High Performance, Q-Sephareose® Big Beads (erhältlich durch die Firma Pharmacia); DEAE-Tris-Acryl, DEAE-Spherodex®, Q-Hyper-D (erhältlich durch die Firma Sepracor); Macroprep® DEAE, Macroprep Q® (Firma BioRad); DEAE-Toyopearl®, QAE-Toyopearl®, Toyopearl® Super-Q (Firma Tosohaas), Protein PAK DEAE (Waters); Fractogel® EMD-TMAE, Fractogel® EMD-DEAE, Fractogel® EMD-DMAE, Licrospher® 1000 TMAE, Licrospher® 1000 DEAE und Licrospher® 4000 DMAE (Firma Merck),vor. Eine besonders bevorzugte Ausführungsform betrifft die Isolierung des Faktor VIIa durch hydrophobe Chromatographie, z.B. unter Verwendung von folgenden Materialien Butyl-Sepharose®, Octyl-Sepharose®, Phenyl-Sepharose®, Phenyl-Sepharose® Fast Flow High Sub, Phenyl-Sepharose® Fast Flow Low Sub, Phenyl-Sepharose® High Performance (alle erhältich von Pharmacia); Fractogel® TSK-Butyl (Firma Merck); Macroprep®-Methyl-HIC-Support, Macroprep® t-Butyl-HIC Support (Firma BioRad); TSK-Gel Butyl Toyopearl®, TSK-Gel Phenyl Toyopearl® und TSK-Gel Ether Toyopearl (Firma Tosohaas). Bei der Reinigung der Gerinnungsfaktoren IX und X ist genauso wie bei dem Gerinnungsfaktor VII eine gleichzeitige Aktivierung - zumindest teilweise - während der Isolierung vorteilhaft. Jedoch ist darauf zu achten, dass die Präparation keine thrombogene Aktivität aufweist; ein Gehalt an aktiviertem Faktor II ist daher zu vermeiden.

Die erfindungsgemässe Zusammensetzung weist vorteilhafterweise eine FEIB-Aktivität im Bereich von 1 - 1000 Einheiten FEIBA/ml gebrauchsfertige Lösung auf. Vorzugsweise beträgt die FEIB-Aktivität mehr als 5 E/ml, am meisten bevorzugt ist eine FEIBA im Bereich von 10 bis 100 E/ml.

Die Erfindung umfasst auch die Verwendung einer Kombination von Faktor VIIa und mindestens einem Wirkstoff zur Herstellung einer pharmazeutischen Zuammensetzung mit FEIB-Aktivität zur raschen Unterbrechung von Blutungen bei Patienten mit einem Gerinnungsfaktor-Mangel. Insbesondere wird bei dieser Verwendung eine rasche Unterbrechung von Blutungen erzielt, welche durch Inhibitoren von Gerinnungsfaktoren bedingt ist.

Die in vivo Wirkung der erfindungsgemässen pharmazeutischen Zusammensetzung lässt sich an einem Tiermodell zeigen, bei welchem Kaninchen nach Behandlung mit einem Faktor VIII-Inhibitorplasma temporär in den Zustand einer Hämophilie A versetzt werden. In derart vorbehandelten Tieren, die eine der Inhibitor-Haemophilie entsprechenden haemorrhagische Diathese aufweisen, lässt sich das abnorme Blutungsverhalten durch die Gabe von 75 bis 150 Einheiten FEIBA/kg auf das eines unbehandelten Kaninchens korrigieren. Um die verbesserte Wirksamkeit einer FEIBA-Präparation mit einem definiert höheren Faktor VIIa-Gehalt zu zeigen, wird eine erfindungsgemässe pharmazeutische Präparation, enthaltend FEIBA und Faktor VIIa in einem Verhältnis von 10 Einheiten Faktor VIIa pro Einheit FEIBA, in einer Dosis von 5 E FEIBA/kg (entsprechend 50 E Faktor VIIa/kg) den blutenden Kaninchen gegeben. Die Blutungsintensität wird dadurch im gleichen Ausmass reduziert wie durch die Gabe von 75 E FEIBA/kg. Als Kontrollen werden konventionelle FEIBA in einer Dosis von 5E/kg oder Faktor VIIa in einer Dosis von 50 E/kg gegeben, die jeweils zu keiner nennenswerten Reduktion der Blutungsintensität im Verhältnis zum unbehandelten Haemophilie A-Inhibitor-Tier führen. Somit ist eine unerwartet hohe Wirksamkeit der erfindungsgemässen pharmazeutischen Zusammensetzung dokumentiert. Ein wesentlicher Vorteil dabei ist, dass das thrombogene Potential der erfindungsgemässen pharmazeutischen Zusammensetzung gegenüber der herkömmlicher FEIBA deutlich reduziert ist.

Die Erfindung wird durch die nachfolgenden Beispiele noch näher beschrieben.

### Beispiele 1 bis 8:

### Verfahren zur Herstellung einer Faktor VIIa-Präparation

### Beispiel 1: Abtrennung von Kryopräzipitat und FEIBA aus Plasma

Die Herstellung erfolgt basierend auf AT-350 726 und AT-368 883.

Frisch gefrorenes, menschliches Citratplasma wurde bei 0 - +4°C aufgetaut und das dabei anfallende Kryopräzipitat durch Zentrifugation bei +2°C abgetrennt. Dem daraus resultierenden Kryoüberstand wurden bei nativem pH-Wert 0,5 g DEAE-Sephadex® A-50 bei +4°C unter ständigem Rühren zugesetzt. Die Suspension wurde eine weitere Stunde gerührt und anschliessend stehen gelassen, so dass der Protein-DEAE-Sephadex®-Komplex sedimentierte. Dabei wurde FEIBA generiert und zusammen mit den Faktoren des Prothrombinkomplexes (II, VII, IX und X) und Inertprotein an DEAE-Sephadex® adsorbiert. Der DEAE-Sephadex®-Protein-Komplex wurde nach abgeschlossenem Adsorptionsvorgang durch Zentrifugation oder Filtration vom Überstand abgetrennt. FEIBA wurde, wie in AT368883 beschrieben, durch Waschung und Elution des DEAE-Sephadex®-Protein-Komplexes gewonnen. Der Überstand wurde zur Faktor VIIa-Präparation weiterverarbeitet.

### Beispiel 2: Abtrennung von Faktor VII/VIIa aus FEIBA-Überstand

Prothrombinkomplexfaktoren, insbesondere Faktor VII und VIIa, wurden nun durch Adsorption an Aluminiumhydroxid aus dem Überstand nach FEIBA-Abtrennung aus Beispiel 1 gewonnen. Dazu wurden pro 1 Liter FEIBA-Überstand 10 ml 2%iger Aluminiumhydrogelsuspension zentrifugiert (SORVALL RC3B, Rotor H6000A, 5000 rpm, 10 Minuten, ca. 4°C). Der daraus gewonnene Niederschlag wurde mit der zu behandelnden FEIBA-Überstandsmenge homogen vermengt (Ultra Turrax). Anschliessend wurde 30 Minuten bei 20-22°C gerührt und danach zentrifugiert (SORVALL RC3B, Rotor H6000A, 5000 rpm, 10 Minuten, ca. 4°C).

Der abzentrifugierte Niederschlag wurde mit 3,5 % des Volumens des zur Adsorption verwendeten FEIBA-Überstandes in einer Lösung von 4 g Na₃Citrat.2H₂O/l und 7 g NaCl/l, pH 7,5, suspendiert und 30 Minuten zur Entfernung von Protein gerührt. Danach wurde der Niederschlag durch Zentrifugieren abgetrennt (RC3B, Rotor H6000A, 5000 rpm, 10 Minuten, ca. 4°C). Der Überstand wurde verworfen und der Niederschlag zur Weiterverarbeitung verwendet. Anschliessend erfolgte ein Schritt zur Inaktivierung möglicherweise vorhandener pathogener Substanzen gemäss AT-A1547/93. Tween® 80 wurde in einer Menge von 1,5 Vol% des zur Adsorption verwendeten FEIBA-Überstandes vorgelegt und auf 55°C erwärmt. Der Niederschlag nach der 1. Waschung wurde in der Tween® 80 Lösung mit Hilfe eines UltraTurrax-Mixers während 1-2 Minuten suspendiert und 10 Minuten bei 55°C gerührt. Danach wurde sofort mit dem 9-fachen Volumen Wasser (+4°C) verdünnt.

Der behandelte Al(OH)₃-Protein-Komplex wurde durch Zentrifugation abgetrennt, der Überstand verworfen und der Niederschlag weiterverarbeitet. Anschliessend erfolgten zwei Waschungen mit je 3,5 Vol% des eingesetzten FEIBA-Überstandes mit Citratpuffer (4 g Na₃Citrat.2H₂O/l und 7 g NaCl/l, pH 7,5) durch Resuspendieren und erneutes Zentrifugieren.

Die den Faktor VII/VIIa enthaltende Fraktion wurde durch Elution mit Phosphatpuffer vom Aluminiumhydroxidgel abgetrennt. Der Protein-Aluminiumhydroxid-Komplex wurde mit 1 Vol % des zur Adsorption verwendeten FEIBA-Überstandes eines 0,3 M Phosphat-puffers, pH 8,6 (53,4 g Na₂HPO₄.2H₂O/l wurden mit einer Lösung von 41,4 g NaH₂PO₄.H₂O/l auf pH 8,6 eingestellt) 30 Minuten gerührt. Anschliessend wurde die feste Phase durch Zentrifugation bei 5000 rpm 10 Minuten bei 20 - 22°C abgetrennt. Der Überstand enthielt Faktor VII/VIIa und wurde zur weiteren Reinigung weiterverarbeitet.

### Beispiel 3: Aktivierung von Faktor VII und Ionenaustauscherreinigung von Faktor VIIa

Das in Beispiel 2 gewonnene Eluat wurde mit destilliertem Wasser im Verhältnis 1 : 1 verdünnt, mit 0,25 mM CaCl₂ versetzt und der pH-Wert auf 8,6 eingestellt. Die Lösung wurde anschliessend mit 65 ml/l mit Puffer vorgewaschener Q-Sepharose FF versetzt und 2 Stunden bei 4°C gerührt. Anschliessend wurde das Gel, an welchem Faktor VIIa nun gebunden vorlag, durch Filtration oder Zentrifugation abgetrennt. Das beladene Gel wurde durch 15 minütiges Resuspendieren und anschliessendes Abtrennen eines Waschpuffers (96,7 g Na₂HPO₄.2H₂O/l und 0,0368 g CaCl₂2H₂O/l wurden mit einer Lösung von 20,7 NaH₂PO₄.H₂O/l auf pH 8,6 eingestellt) vom Inertprotein befreit. Anschliessend wurde Faktor VIIa durch Elution mit einer Lösung von 105,7 g (NH₄)₂SO₄/l, 58,5 g NaCl/l und 2,42 g TrisHCl/l, pH 7,4, durch 30 minütige Suspension eluiert. Der den Faktor VIIa enthaltende Gelüberstand wurde durch Filtration oder Zentrifugation abgetrennt.

### Beispiel 4: Reinigung von Faktor VIIa durch hydrophobe Chromatographie

Die den Faktor VIIa enthaltende Lösung aus Beispiel 3 wurde über Phenylsepharose LS chromatographisch gereinigt. Eine Säule mit 50 mm ID und 24 mm Betthöhe wurde mit Phenylsepharose LS gefüllt und in einem Puffer enthaltend 2,42 g TrisHCl/l und 105,7 g (NH₄)₂SO₄/l, pH 7,4 äquilibriert. 100 ml des Faktor VIIa enthaltenden Eluates aus Beispiel 3 wurden mit einer Flussrate von 23 ml/min. über das Gel gepumpt. Dabei wurde Faktor VIIa an das Gel gebunden, Inertprotein befand sich im Durchlauf. Das Gel wurde mit dem zur Äquilibrierung verwendeten Puffer nachgewaschen. Anschliessend wurde mit einer Lösung von 2,42 g TrisHCl/l, pH 7,4, Faktor VIIa eluiert. Dabei wurden Fraktionen gesammelt. Die Faktor VIIa-Aktivität enthaltenden Fraktionen wurden vereinigt und durch Chromatographie über Sephadex G-25 gegen einen Puffer, enthaltend 2,42 g TrisHCl/l, pH 7,4, umgepuffert.

### Beispiel 5: Reinigung von Faktor VIIa durch Chromatographie an Q-Sepharose®

Q-Sepharose® FF, gepackt in eine Säule mit 25 mm ID und 55 mm Betthöhe, wurde mit 20 mM TrisHCl/l, pH 7,4 äquilibriert. Anschliessend wurden 43 ml der Faktor VIIa enthaltenden Lösung aus Beispiel 4 bei einer Flussrate von 5,3 ml/min. über die Säule gepumpt. Dabei wurde Faktor VIIa an das Gel gebunden während Inertprotein im Durchlauf bleibt. Anschliessend wurde mit einem Puffer, enthaltend 25 mM Na₃Citrat.2H₂O und 80 mM NaCl, pH 6,0, nachgewaschen. Dabei wurde weiteres Inertprotein abgetrennt. Die Elution von Faktor VIIa erfolgte durch Spülen der Säule mit 25 mM Na₃Citrat.2H₂O und 160 mM NaCl, pH 6,0. Die Protein und Faktor VIIa-Aktivität enthaltenden Fraktionen dieser Elutionsstufe wurden gepoolt. Anschliessend wurde die Säule mit einem Puffer (25 mM Na₃Citrat.2H₂O und 1M NaCl, pH 6,0) regeneriert und wieder verwendet.

### Beispiel 6: Ultrakonzentration der Faktor VIIa-Präparation

Der Faktor VIIa enthaltende Pool aus Beispiel 5 wurde auf pH 7,0 eingestellt und mit 0,1 % Humanalbumin versetzt. Anschliessend wurde über Ultrafiltrationsmembranen (AMICON YM10, cut-off 10.000 D) unter einem Druck von 3,0 bar in einer AMICON 80/50 Rührzelle auf ¹/₁₀ des Ausgangsvolumens eingeengt. Die konzentrierte, Faktor VIIa enthaltende Lösung wurde anschliessend lyophilisiert.

### Beispiel 7: Hitzebehandlung von lyophilisiertem Faktor VIIa

Zur Inaktivierung eventuell enthaltener Krankheitserreger wurde die lyophilisierte Faktor VIIa-Präparation aus Beispiel 6 nach dem im Patent EP0159311 beschriebenen Verfahren unter Erhöhung des partiellen Wasserdampfdruckes erhitzt bzw. im Anschluss an die Behandlung bei 60°C noch eine Stunde bei 80°C ebenso behandelt. Die Ausbeute an Faktor VIIa betrug in jedem Fall mehr als 90%.

### Beispiel 8: Charakterisierung der Faktor VIIa-Präparation

Die Bestimmung des Aktivierungsgrades des Faktor VII wurde nach der von Van Deijk, Haemostasis 13:192-197 (1983), beschriebenen Methode durchgeführt. Der Gerinnungstest basiert auf der Verwendung von einerseits bovinem und andererseits humanem Thromboplastin in Verbindung mit einem Faktor VII-Mangelplasma. Gemessen wurde gegen humanes Normalplasma als Standard. Die spezifischen Aktivitäten an Faktor VIIa für die einzelnen Stufen der Herstellung und Reinigung sowie das Aktivierungsverhältnis, Faktor VII gemessen mit bovinem Thromboplastin gegen Faktor VII gemessen mit humanem Thromboplastin, sind Tabelle 1 zu entnehmen. Die Proteinbestimmungen erfolgten nach der Methode von Bradford, Anal.Biochem. 72:248-254 (1976).

### Beispiel 9: Kombination von FEIBA und Faktor VIIa

Frisch gefrorenes menschliches Citratplasma wurde bei 0 - 4°C aufgetaut und das dabei anfallende Kryopräzipitat durch Zentrifugation bei +2°C abgetrennt. Dem daraus resultierenden Überstand wurden bei nativem pH-Wert 0,5 g DEAE-Sephadex A-50 bei +4°C unter ständigem Rühren zugesetzt. Die Suspension wurde eine weitere Stunde gerührt und anschliessend stehen gelassen, so dass der Protein-DEAE-Sephadex-Komplex sedimentierte. Dabei wurde FEIBA generiert und zusammen mit den Faktoren des Prothrombinkomplexes und Inertprotein an DEAE-Sephadex® adsorbiert. Der DEAE-Sephadex® -Proteinkomplex wurde nach beendetem Adsorptionsvorgang durch Filtration vom Überstand abgetrennt. FEIBA wurde, wie in AT-368 883 beschrieben, durch Waschung mit Puffer und anschliessende Elution mit Kochsalzlösung vom DEAE-Sephadex® desorbiert. Die FEIBA-enthaltende Lösung wurde durch Ultrafiltration auf 1/5 des Ausgangsvolumens eingeengt und anschliessend zur Autoaktivierung der Prothrombinkomplexfaktoren 20 Stunden bei Raumtemperatur inkubiert. Anschliessend wurde die den aktivierten Prothrombinkomplex enthaltende Lösung lyophilisiert. Dann wurde das Pulver auf eine Konzentration von 30 mg Protein/ml in einem Puffer aus 2 g Na₃Citrat.2H₂O und 4 g NaCl, pH 7,2 gelöst. Diese Lösung wurde durch Filter mit einer Porengrösse von 1 µm filtriert und neuerlich lyophilisiert. Das so gewonnene Pulver wurde in einer Faktor VIIa-enthaltenden Lösung, die, wie in Beispiel 4 beschrieben, hergestellt wurde, gelöst. Die Lösung wurde so eingestellt, dass pro 1 E FEIBA, getestet gemäss AT-350 726, 10 E FVIIa enthalten waren. Diese Mischung wurde neuerlich lyophilisiert nach dem Verfahren der EP-0159 311, 10 Stunden bei 60°C und 1 Stunde bei 80°C behandelt. FEIB-Aktivität und die FVIIa-Aktivität des Pulvers wurden vor und nach der Hitzebehandlung bestimmt. Nach dem Hitzebehandlungsschritt betrug die Aktivität von FVIIa in der Präparation 96% des Ausgangsmaterials und hatte somit um 4% abgenommen; die FEIB-Aktivität betrug 90%, entsprechend einem Verlust von 10%.

Als Vergleich dazu wurde die FVIIa-Präparation aus Beispiel 4 lyophilisiert und ebenso hitzebehandelt. Dabei nahm die Aktivität um 48% ab.

### Beispiel 10: Kombination von Prothrombinkomplex und Faktor VIIa

Eine Prothrombinkomplex enthaltende Präparation wurde nach der Methode von H.G.J. Brummelhuis, Methods of Plasma Protein Fractionation, J.M. Curling (Hrsg.), Seite 117-128, Academic Press 1980, hergestellt. Die Präparation enthielt Faktor II, IX und X in ähnlichem Verhältnis. Anschliessend wurde die Präparation mit Faktor VIIa, der wie in Beispiel 4 beschrieben hergestellt wurde, versetzt. Die Mischung wurde so eingestellt, dass pro 1 E FX 10 E FVIIa enthalten waren. Ein gefriergetrocknetes Pulver dieser Mischung wurde nach dem Verfahren der EP-159 311 10 Stunden bei 60°C und 1 Stunde bei 80°C behandelt. Die Aktivitäten der Gerinnungsfaktoren II, IX, X und Faktor VIIa wurden jeweils vor und nach der Hitzebehandlung bestimmt. Die Aktivitätsverluste betrugen für Faktor II 7%, für Faktor IX 12%, für Faktor X 3% und für Faktor VIIa 1%.

Als Vergleich dazu wurde die Faktor VIIa-Präparation aus Beispiel 4 lyophilisiert und ebenso hitzebehandelt. Dabei nahm die Aktivität um 48% ab.

### Beispiel 11: Gewinnung einer Faktor VIIa/FEIBA-Präparation

Eine Faktor VIIa- und FEIB-Aktivität enthaltende Präparation wurde wie folgt gewonnen. Frisch gefrorenes menschliches Citratplasma wurde bei 0 - +4 °C aufgetaut und das dabei anfallende Kryopräzipitat durch Zentrifugation bei +2 °C abgetrennt. Der daraus resultierende Kryoüberstand wurde bei nativem pH-Wert mit 0,5 g QAE-Sephadex A50 (Fa. Pharmacia) versetzt und bei +4 °C 15 Stunden gerührt. Anschliessend wurde der Gel-Protein-Komplex durch Sedimentation und Filtration vom Überstand abgetrennt, und die Faktor VIIa und FEIBA enthaltende Fraktion durch Elution mit 3 %iger NaCl-Lösung gewonnen. Faktor VIIa und FEIBA wurden quantitativ bestimmt. Die Präparation enthielt 12,7 E FVIIa pro Einheit FEIBA.

### Beispiel 12: Gewinnung einer Faktor VIIa/FEIBA-Präparation

FEIBA wurde wie in Beispiel 11 beschrieben gewonnen. Als Ionenaustauscher zur Adsorption wurde allerdings Fractogel® EMD TMAE (Fa. MERCK) in einer Konzentration von 5 ml feuchtem Gel pro Liter Kryoüberstand verwendet. Nach Elution der aktivierten Prothrombinkomplexfaktoren mit 3 %iger NaCl-Lösung enthielt diese 54 E FEIBA/ml sowie 0,6 E Faktor VIIa/E FEIBA. Um das erfindungsgemässe Präparat herzustellen, wurde daher aus dem Kryoüberstand Faktor VIIa gemäss Beispielen 2-4 gewonnen und der FEIBA-haltigen Lösung zugesetzt, so dass schliesslich mindestens 10 E Faktor VIIa/E FEIBA enthalten waren.

### Beispiel 13: Gewinnung einer Faktor VIIa/FEIBA-Präparation

Analog zu Beispiel 12 wurde wiederum FEIBA hergestellt. Als Ionenaustauscher wurde Q-Sepharose Fast Flow (Fa. Pharmacia) in einer Konzentration von 5 ml pro Liter Kryoüberstand verwendet. Die FEIB-Aktivität im erhaltenen Eluat betrug 1.280 E/ml und 0,3 E Faktor VIIa/E FEIBA. Um eine Präparation mit 20 E Faktor VIIa/E FEIBA herzustellen, wurde wie in Beispiel 11 Faktor VIIa aus demselben Kryoüberstand, aus dem die FEIBA-Präparation gewonnen wurde, hergestellt und der FEIBA-haltigen Lösung zugemischt.

### Beispiel 14: In vivo Wirksamkeit einer Faktor VIIa/FEIBA-Präparation

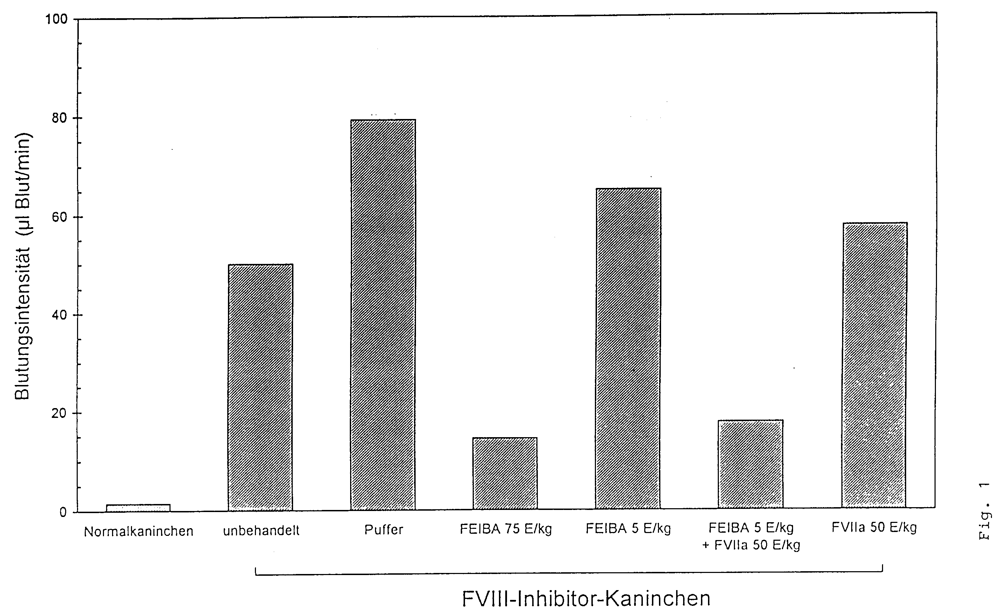

Eine gemäss Beispiel 9 hergestellte Kombination von FEIBA und Faktor VIIa wurde wie folgt in einem Kaninchen mit Faktor VIII-Inhibitor-Haemophilie getestet. Ca. 2 kg schwere weisse Neuseelandkaninchen wurden narkotisiert. Nach Eintritt der Narkose wurde die rechte Femoralvene präpariert und ein permanenter venöser Zugang geschaffen. Durch diesen wurden 0,5 ml/kg Körpergewicht eines humanen Faktor VIII-Inhibitorplasmas (1500 Bethesda Einheiten/ml) über 10 min infundiert. 30 min nach Abschluss der Infusion wurden die Blutungscharakteristiken mit einer modifizierten Methode nach Giles et al, Blood 60:727-730 (1982), bestimmt. Dazu wurde das Fell um eine Kralle zur Hinterpfote des Kaninchens rasiert, um zu verhindern, dass bei der späteren Blutung austretendes Blut vom Fell absorbiert wird. Die Nagelhaut wurde mittels einer Krallenzange verletzt. Unmittelbar danach wurden Filter unterhalb der Wunde so etabliert, dass das Blut direkt auf den Filter tropfen konnte, ohne von diesem durch Kapillarwirkung aufgesogen zu werden. Durch diese Massnahme wurde verhindert, dass ein sich formierendes Blutgerinnsel zerstört wird. Die Filter wurden alle 2 min gewechselt und das austretende Blut in Fraktionen gesammelt. Die Blutsammlung wurde 30 min fortgesetzt. Die Qualifizierung der Blutungscharakteristik erfolgt durch Extraktion des in Fraktionen auf dem Filter gesammelten Blutes mit jeweils 5 ml 0,04 %iger Ammoniumhydroxidlösung über 5 h. Dabei wurden die Erythrozyten lysiert, die mit dem Blut im Filter gesammelt wurden. Durch eine 10-minütige Ultraschallbehandlung wurde das Haemoglobin extrahiert und quantitativ photometrisch bei 416 nm gegen eine Eichkurve bestimmt, wobei die Eichkurve dadurch ermittelt wurde, dass Kaninchenblutvolumina zwischen 10µl und 1 ml auf die Filter pipettiert, diese, wie oben beschrieben, extrahiert wurden und das Haemoglobin photometrisch bei 416 nm bestimmt wurde. Entsprechend liessen sich lineare Eichkurven erstellen, die eine direkte Umrechnung der Haemoglobinkonzentration auf die Blutmenge pro Filter ermöglichten. Die Blutungscharakteristik des Nagelschnittes wurde ermittelt, indem der kumulierte Blutverlust durch additives graphisches Auftragen der einzelnen Blutfraktionen gegen die Zeit erfolgte. Als für die Blutung relevantes Kriterium wurde die Steigung der kumulierten Blutung zwischen 10 und 20 min des Versuches herangezogen und diente als Mass für die Blutungsintensität. Die mittlere Blutungsintensität eines derart behandelten Tieres ist aus der Abbildung (Fig.1) zu entnehmen.

Zur Testung der Wirksubstanzen wurde nach herbeigeführter Inhibitorhaemorrhagie des Kaninchens eine Lösung einer pharmazeutischen Zubereitung, enthaltend den jeweiligen Wirkstoff in einem Volumen von 30 ml mit einer Infusionsgeschwindigkeit von 1 ml/min kontinuierlich infundiert und gleichzeitig mit dem Beginn der Infusion die Blutungsintensität neuerlich, wie oben beschrieben, ermittelt. Entsprechend wurde eine FEIBA-Präparation, hergestellt gemäss AT-350 726 oder AT-368 883, in einer Dosis von 75 E FEIBA/kg an ein entsprechend vorbehandeltes Kaninchen verabreicht. Diese Dosis führte zu einer drastischen Reduktion der Blutungsintensität (s. Fig. 1).

Als Kontrolle wurde eine Pufferlösung ohne Wirkstoff infundiert, die zu keiner Reduktion der Blutung führte. Die Gabe von FEIBA in einer Dosis von 5 E/kg führte ebenso zu keiner signifikanten Reduktion der Blutungsintensität.

Wenn hingegen eine erfindungsgemässe Präparation entsprechend einer Dosis von 5 E FEIBA/kg und 50 E Faktor VIIa/kg dem Kaninchen gegeben wurde, kam es zu einer Reduktion der Blutungsintensität, vergleichbar mit einer effektiven FEIBA-Dosis, während 50 E Faktor VIIa/kg keinen Effekt auf die abnorm erhöhte Blutungsintensität haben.

**Tabelle 1**

| Reinigung von FVIIa, Analyse der Zwischenprodukte | | | | | |
|---|---|---|---|---|---|
| | **FVII-Gerinnung** | | **FVIIa-Gerinnung** | | |
| | Thromboplastin human | | Thromboplastin bovin | | bovin/human |
| **Stufe** | spez. Aktivität E/mg | Reinigungsfaktor | spez. Aktivität E/mg | Reinigungsfaktor | Aktivierungsgrad |
| FEIBA-Überstand | 0,013 | 1 | 0,018 | 1 | 1,26 |
| Aluminiumhydroxyd-Eluat | 14,4 | 1068 | 62,9 | 3559 | 4,17 |
| Q-Sepharose-Aktivierung | 89,0 | 6591 | 447,8 | 25338 | 4,78 |
| Phenylsepharose Pool | 276,0 | 20448 | 1716,4 | 97128 | 6,59 |
| Q-Sepharose Pool | 820,2 | 60757 | 5408,3 | 306057 | 6,95 |
| Ultrakonzentrat (mit Alb.) | 4,6 | | 29,5 | | |
| Lyophilisat | 4,0 | | 42,2 | | |
| 10Std/60°C | 4,1 | | 46,7 | | |
| 10Std/60°C+1Std/80°C | 4,2 | | 36,2 | | |

## Patentansprüche

1. Pharmazeutische Zusammensetzung mit FEIB-Aktivität zur Behandlung von Patienten mit Blutgerinnungsstörungen, die durch einen Gerinnungsfaktor-Mangel bzw. Inhibitoren von Gerinnungsfaktoren bedingt sind, dadurch gekennzeichnet, dass sie den Faktor VIIa und mindestens einen weiteren Wirkstoff enthält und die Aktivität von mindestens 10 Faktor VIIa-Einheiten pro Einheit FEIBA aufweist.

2. Pharmazeutische Zusammensetzung nach Anspruch 1, dadurch gekennzeichnet, dass sie eine Aktivität im Bereich von 10 bis 100 Faktor VIIa-Einheiten pro Einheit FEIBA aufweist.

3. Pharmazeutische Zusammensetzung nach Anspruch 2, dadurch gekennzeichnet, dass sie eine Aktivität im Bereich von 10 bis 20 Faktor VIIa-Einheiten pro Einheit FEIBA aufweist.

4. Pharmazeutische Zusammensetzung nach Anspruch 2, dadurch gekennzeichnet, dass sie eine Aktivität im Bereich von 10 bis 15 Faktor VIIa-Einheiten pro Einheit FEIBA aufweist.

5. Pharmazeutische Zusammensetzung nach Anspruch 1 bis 4, dadurch gekennzeichnet, dass neben Faktor VIIa als weitere Wirkstoffe die Gerinnungsfaktoren II, IX und X, vorzugsweise in einem Verhältnis von jeweils 0,5 bis 2 Einheiten pro Einheit FEIBA, enthalten sind.

6. Pharmazeutische Zusammensetzung nach Anspruch 1 bis 4, dadurch gekennzeichnet, dass neben Faktor VIIa als weitere Wirkstoffe die Gerinnungsfaktoren II, IX und X, vorzugsweise in einem Verhältnis von jeweils 0,5 bis 1,5 Einheiten pro Einheit FEIBA, enthalten sind.

7. Pharmazeutische Zusammensetzung nach Anspruch 1 bis 6, dadurch gekennzeichnet, dass sie im weiteren Protein C und/oder Protein S umfasst.

8. Pharmazeutische Zusammensetzung nach Anspruch 1, dadurch gekennzeichnet, dass sie aus einer Fraktion, welche den gereinigten Prothrombinkomplex enthält, und einer Fraktion, welche den gereinigten Faktor VIIa enthält, zusammengesetzt, wobei die spezifische Aktivität des Faktor VIIa > 10 E/mg Protein beträgt, und zur Inaktivierung von Viren behandelt ist.

9. Pharmazeutische Zusammensetzung nach Anspruch 1, dadurch gekennzeichnet, dass sie aus einer Fraktion, welche den gereinigten partiellen Prothrombinkomplex enthält, und einer Fraktion, welche den gereinigten Faktor VIIa enthält, zusammengesetzt, wobei die spezifische Aktivität des Faktor VIIa > 10 E/mg Protein beträgt, und zur Inaktivierung von Viren behandelt ist.

10. Pharmazeutische Zusammensetzung nach Anspruch 8 oder 9, dadurch gekennzeichnet, dass der Prothrombinkomplex als aktivierter Prothrombinkomplex oder FEIBA-Präparation vorliegt.

11. Pharmazeutische Zusammensetzung nach Anspruch 8 oder 9, dadurch gekennzeichnet, dass die den Prothrombinkomplex sowie den Faktor VIIa enthaltenden Faktoren von demselben Plasmapool stammen.

12. Verfahren zur Herstellung einer pharmazeutischen Zusammensetzung nach einem oder mehreren der Ansprüche 1 bis 7, gekennzeichnet durch
- Kontaktieren einer Fraktion, welche den Gerinnungsfaktor VII enthält, mit einem Anionenaustauscher, wobei der Gerinnungsfaktor VII zumindest teilweise aktiviert wird,
- Abtrennen des Anionenaustauschers und Gewinnen der Fraktion, enthaltend den Gerinnungsfaktor VIIa, wobei die Fraktion vor dem Kontaktieren eine FEIBA besitzt bzw. währenddessen eine FEIBA in der Fraktion generiert wird.

13. Verfahren nach Anspruch 12, dadurch gekennzeichnet, dass Anionenaustauscher mit quaternären Ammoniumgruppen, z. B. vom QAE-, TMAE- und Q-Typ eingesetzt werden.

14. Verfahren zur Herstellung einer pharmazeutischen Zusammensetzung mit FEIB-Aktivität nach einem der Ansprüche 8 bis 10, welches die folgenden Schritte umfasst:
a) Isolieren der Gerinnungsfaktoren II, IX und X als Einzelkomponenten oder als Gemisch aus einem oder mehreren Ausgangsmaterialien, welche(s) diese Gerinnungsfaktoren enthalten (enthält), und
b) Isolieren des Gerinnungsfaktors VII/VIIa aus einem Ausgangsmaterial, welches den Gerinnungsfaktor VII enthält, wobei der Gerinnungsfaktor VII zumindest teilweise zu VIIa aktiviert wird, und
c) entweder die genannten Ausgangsmaterialien oder die jeweiligen Gerinnungsfaktoren zur Inaktivierung von infektiösen Agenzien vorbehandelt bzw. behandelt werden, und
d) Mischen der isolierten Gerinnungsfaktoren mit einem pharmazeutisch akzeptablen Träger.

15. Verfahren nach Anspruch 14, dadurch gekennzeichnet, dass die Gerinnungsfaktoren aus einem einzigen Ausgangsmaterial, vorzugsweise einem Plasmapool oder einer Plasmafraktion, isoliert werden.

16. Verfahren nach Anspruch 14, dadurch gekennzeichnet, dass zur Inaktivierung von infektiösen Agenzien eine Hitzebehandlung durchgeführt wird.

17. Verfahren nach Anspruch 16, dadurch gekennzeichnet, dass eine Hitzebehandlung der Gerinnungsfaktoren in festem Zustand durchgeführt wird.

18. Verfahren nach Anspruch 16, dadurch gekennzeichnet, dass die Hitzebehandlung in Abwesenheit von Albumin erfolgt.

19. Verfahren nach einem oder mehreren der Ansprüche 14 bis 18, dadurch gekennzeichnet, dass die Behandlung zur Inaktivierung von infektiösen Agenzien nach dem Zusammenmischen der Gerinnungsfaktoren mit dem pharmazeutisch akzeptablen Träger durchgeführt wird.

20. Verfahren nach einem oder mehreren der Ansprüche 14 bis 19, dadurch gekennzeichnet, dass der Gerinnungsfaktor VIIa durch hydrophobe Chromatographie isoliert wird.

21. Verfahren nach Anspruch 14 bis 19, dadurch gekennzeichnet, dass die Gerinnungsfaktoren IX, X sowie VII während der Isolierung zumindest teilweise aktiviert werden.

22. Verwendung einer Kombination von Faktor VIIa und mindestens einem weiteren Wirkstoff zur Herstellung einer pharmazeutischen Zusammensetzung mit FEIB-Aktivität nach einem der Ansprüche 1 bis 11 zur raschen Unterbrechung von Blutungen bei Patienten mit einem Gerinnungsfaktor-Mangel.

23. Verwendung nach Anspruch 22, wobei die Präparation zur raschen Unterbrechung von Blutungen, bedingt durch Inhibitoren von Gerinnungsfaktoren, geeignet ist.
